# EUROPÄISCHE PATENTSCHRIFT

(11) **EP 0 764 847 B1**
(45) Veröffentlichungstag und Bekanntmachung des Hinweises auf die Patenterteilung: **31.07.2002**
(21) Anmeldenummer: 95114766.9
(22) Anmeldetag: 20.09.1995
(51) Int. Cl.: G01N 33/00, G01N 27/16

(54) **Gasdetektor**
Gas sensor
Capteur de gaz

(43) Veröffentlichungstag der Anmeldung: 26.03.1997
(73) Patentinhaber: Siemens Building Technologies AG, 8034 Zürich (CH)
(72) Erfinder: Forster, Martin, Dr., CH-8645 Jona (CH); Witikovsky, Friedrich, CH-8604 Volketswil (CH); Scherrer, Niklaus, CH-8645 Jona (CH); Honegger, Susanne, CH-8610 Uster (CH)
(74) Vertreter: Dittrich, Horst, Dr.

(56) Entgegenhaltungen:
- EP-A- 0 016 351
- EP-A- 0 032 844
- GB-A- 1 057 735
- GB-A- 2 088 559
- US-A- 3 476 517
- PATENT ABSTRACTS OF JAPAN vol. 007 no. 266 (P-239) ,26.November 1983 & JP-A-58 146845 (RICOH KK;OTHERS: 01) 1.September 1983,
- PATENT ABSTRACTS OF JAPAN vol. 009 no. 221 (P-386) ,7.September 1985 & JP-A-60 080749 (SHIN COSMOS DENKI KK) 8.Mai 1985,

## Beschreibung

Die vorliegende Erfindung betrifft einen Gasdetektor, mit einem Metallgehäuse, mit einem aufheizbaren Gassensor und mit einer diesen abdeckenden Metallkappe mit einem aus zwei Lagen bestehenden Metallgitter, welches den Gassensor gegen die Umgebung abschirmt.

Da der Gassensor derartiger Gasdetektoren auf bis zu 300° C aufgeheizt wird, wirkt er im Fall des Vorhandenseins von brennbaren Gasen oder Rauchgasen in der zu untersuchenden Luft in einer Konzentration oberhalb der Explosionsgrenze als Zünder und es kommt innerhalb des Detektorgehäuses zu einer Explosion. Das doppellagige Metallgitter dient nun dazu, zu verhindern, dass die Explosionswärme weitergeleitet und das Gas auch ausserhalb des Detektors gezündet wird. Das Metallgitter nimmt die Wärme auf und leitet sie an die umgebende Kappe ab. Selbstverständlich muss das Gitter in der Kappe ausreichend gut verankert sein, damit es bei der genannten Explosion nicht aus der Kappe herausgeschleudert wird.

Bei den heute erhältlichen Gasdetektoren der genannten Art sind zwei übereinander liegende Gitter vorgesehen, die mit der Kappe verschweisst sind, wobei beim Schweissvorgang eine Schweisselektrode die Kappe und die andere das Gitter kontaktiert. Es hat sich gezeigt, dass die beiden dicht aufeinander aufliegenden Gitterlagen in der Regel gegeneinander verschoben sind, wobei diese Verschiebung so weit gehen kann, dass sich die beiden Gitterlagen gegenseitig abdecken. Ausserdem führt der direkte Kontakt zwischen dem Gitter und der einen Schweisselektrode zu einer starken Abnützung der letzteren, weil sich auf die aus einem relativ weichen Material, wie beispielsweise Kupfer, bestehende Schweisselektrode die Gitterstruktur aufprägt.

Es ist offensichtlich, dass die gegenseitige Verschiebung der beiden Gitterlagen nachteilig ist, weil dadurch von Detektor zu Detektor die Diffusion der Gase durch das Gitter und damit die Ansprechzeit der einzelnen Detektoren stark verschieden sind. Ebenso ist offensichtlich, dass die Abnützung der das Gitter kontaktierenden Schweisselektrode nachteilig ist, weil diese Elektrode in relativ kurzen Abständen ausgewechselt werden muss, was unerwünschte Produktionsunterbrechungen bedeutet.

Durch die Erfindung sollen nun die bekannten Gasdetektoren so verbessert werden, dass die genannte Überdeckung der Gitteröffnungen nicht mehr auftritt und die Abnützung der das Gitter kontaktierenden Schweisselektrode vermieden wird.

Diese Aufgabe wird erfindungsgemäss dadurch gelöst, dass an der dem Gassensor zugewandten Seite des Gitters dessen Rand durch einen Ring abgedeckt und dass der Ring über das Gitter mit der Kappe verschweisst ist.

Praktische Versuche haben gezeigt, dass bei der Produktion des erfindungsgemässen Gasdetektors keine Abnützung der einen Schweisselektrode mehr stattfindet, dass die Gitter in der Kappe ausserordentlich stark halten, und dass der nachteilige Überdekkungseffekt der beiden Gitterlagen nicht mehr auftritt. Letzteres deswegen, weil nach dem Schweissvorgang die innere Gitterlage eben bleibt, wogegen sich die äussere nach aussen wölbt. Dadurch berühren sich die beiden Gitterlagen praktisch nur noch in ihrem unterhalb des Ringes liegenden schmalen Randbereich.

Der Ausdruck Gasdetektor ist in der vorliegenden Beschreibung umfassend zu verstehen und bezeichnet sowohl Detektoren zum Nachweis brennbare Gase, wie beispielsweise Propan, Butan oder Kohlenmonoxid, als auch Detektoren zum Nachweis organischer Brandgase und Brandaerosole. Denn auch die letzteren Detektoren (siehe dazu beispielsweise die US-A-4,176,311) weisen eine erhitzte Metall- oder Halbleiteroberfläche auf, die auf ein entsprechendes Gasgemisch als Zünder wirken kann, so dass auch hier eine Abschirmung der erhitzten Oberfläche durch ein Gitter erfolgen muss.

Im folgenden wird die Erfindung anhand eines Ausführungsbeispiels und der Zeichnungen näher erläutert; es zeigt:
- Fig. 1: eine perspektivische Darstellung eines erfindungsgemässen Gasdetektors mit teilweise aufgeschnittener Kappe,
- Fig. 2: einen Längsschnitt durch die Kappe des Detektors von Fig. 1 beim Schweissvorgang,
- Fig. 3: ein schematisches Detail der Kappe von Fig. 2; und
- Fig. 4: die Kappe von Fig. 2 nach dem Schweissvorgang.

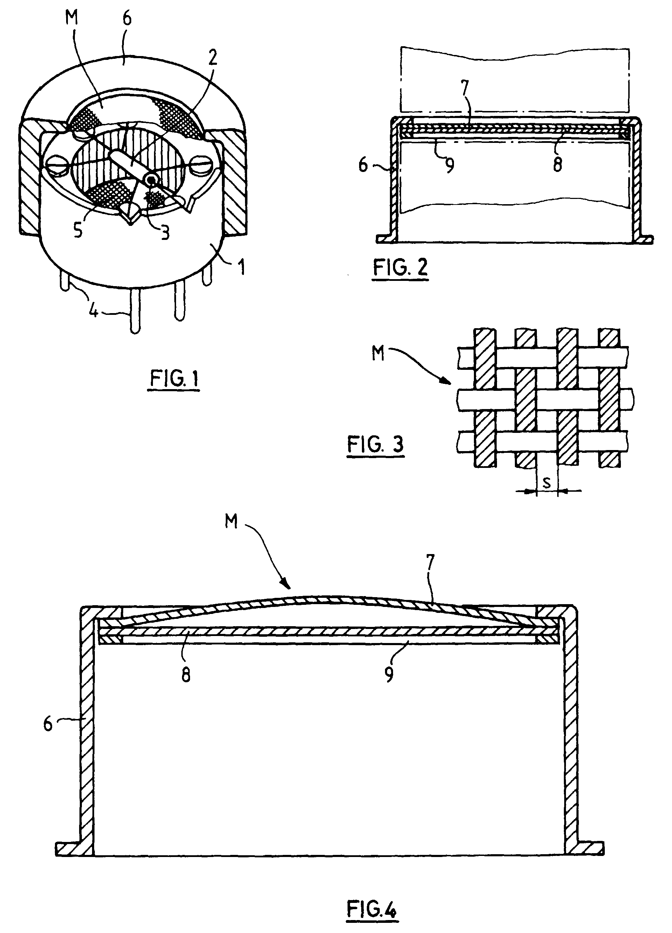

Der in Fig. 1 dargestellte Gasdetektor dient zum Nachweis von explosiven und/oder toxischen Gasen; er weist beispielsweise gegenüber Propan, Butan und Kohlenmonoxid eine hohe Empfindlichkeit auf. Der Detektor besteht aus einem zylindrischen, oben und unten offenen Gehäuse 1 aus Metall, an dessen oberer Stirnseite zwei zu zwei Elektroden (nicht dargestellt) führende Gruppen von je drei Kontakten vorgesehen sind. An je zwei Kontakte jeder Gruppe ist ein Gassensor 2, beispielsweise ein Halbleitersensor, angeschlossen, der dritte Kontakt jeder Gruppe ist mit einer zur Erhitzung des Gassensors 2 vorgesehenen Heizwendel 3 verbunden. Mit dem Bezugszeichen 4 sind unten vom Gehäuse 1 wegragende Steckerstifte bezeichnet. Die bodenseitige Öffnung des Gehäuses 1 ist durch ein feuerfestes doppellagiges Stahlgitter 5 aus rostfreiem Stahl abgedeckt, die obere Öffnung mit dem Gassensor 2 durch eine Kappe 6, die ebenfalls aus Metall gefertigt und mit dem Gehäuse 1 fest verbunden ist. Diese Verbindung erfolgt beispielsweise durch Umbördeln des unteren Randes der Kappe 6 nach dem Aufschieben auf das Gehäuse 1.

Gemäss den Figuren 1 und 2 hat die Kappe 6 die Form eines Topfes mit einem bis auf einen relativ schmalen Rand offenen Boden. Auf diesen Boden ist innen ein aus zwei Lagen 7 und 8 bestehendes Metallgitter M aus rostfreiem Stahl gelegt, welches die Öffnung des Bodens abdeckt. Das Gitter M, das den Gassensors 2 nach aussen abschirmt, soll verhindern, dass es ausserhalb des Gasdetektors zu einer Explosion kommt. Denn der durch die Heizwendel 3 aufgeheizte Gassensor 2 wirkt im Fall des Vorhandenseins von brennbaren Gasen in der zu untersuchenden Luft in einer Konzentration oberhalb der Explosionsgrenze auf diese Gase als Zünder, so dass es innerhalb des Gehäuses 1 zu einer Explosion kommt. Das doppellagige Metallgitter M nimmt die Wärme auf und leitet sie an die umgebende Kappe 6 ab und verhindert dadurch eine Weiterleitung der Wärme nach aussen. Um an den Gassensor 2 zu gelangen, muss die zu untersuchende Luft durch das Metallgitter M diffundieren, dessen Geometrie das Diffusionsverhalten der Luft und damit die Ansprechzeit des Detektors wesentlich beeinflusst. Die Geometrie des Gitters M sollte daher möglichst immer die gleiche sein.

Fig. 3 zeigt einen schematischen Auschnitt aus dem Metallgitter M, wobei davon ausgegangen wird, dass die beiden Lagen 7 und 8 des Gitters exakt fluchten. Bei dieser Relativposition der beiden Gitterlagen und unter der Annahme, dass die Maschenweite s des Gitters gleich der halben Gitterperiode ist, ist die Öffnung im Boden des Deckels 6 durch das Metallgitter M zu 50% abgedeckt. Wenn sich nun die beiden Gitterlagen 7 und 8 relativ zueinander verschieben, denn wird die Durchlassöffnung des Gitters M abnehmen und kann bei einer Relatiwerschiebung um die Maschenweite s praktisch null werden, was eine drastische Erhöhung der Ansprechzeit des betreffenden Detektors bedeuten würde.

So unerwünscht und nachteilig dieser Einfluss der Relativposition der beiden Lagen 7 und 8 des Metallgitters M auf die Ansprechzeit des Detektors auch ist, so ist die Verwendung eines aus zwei Lagen bestehenden Gitters notwendig. Es ist aber praktisch nicht möglich, die Kappe 6 mit einem vertretbaren Aufwand so zu produzieren, dass die beiden Gitterlagen 7 und 8 eine definierte Relativposition einnehmen.

Beim erfindungsgemässen Gasdetektor wird nun eine Lösung vorgeschlagen, die eine kostengünstige Produktion von Detektoren mit gleicher Ansprechzeit ermöglicht. Diese Lösung besteht gemäss den Figuren 2 und 4 darin, dass vor dem Verschweissen des Gitters M mit der Kappe 6, welches durch zwei in Fig. 2 strichpunktiert angedeutete Elektroden erfolgt, auf das Gitter M ein Ring 9 in der Art einer Unterlagscheibe gelegt und dann die eine Elektrode gegen diesen Ring gedrückt wird. Beim Schweissvorgang wirken die beiden Lagen 7 und 8 des Gitters M wie Schmelzlot, wodurch Gitter und Ring im Bereich ihres Randes mit der Kappe 6 verschweisst werden. Der Ring 9 besteht vorzugsweise aus dem gleichen Material wie das Gitter M, beim beschriebenen Beispiel also aus rostfreiem Stahl, und er weist eine Dicke von 0,1 bis 0,3 mm auf. Es ist aber auch möglich, für Ring und Gitter verschiedene Materialien einzusetzen.

In Fig. 4 ist die Kappe 6 nach dem Schweissvorgang dargestellt. Wie man der Figur entnehmen kann, befindet sich die innere Lage 8 des Gitters M in ihrer ursprünglichen ebenen Position, wogegen die äussere Lage 7 nach aussen gewölbt ist. Somit besteht zwischen den beiden Lagen 7 und 8 des Gitters M ein so grosser Abstand, dass eine eventuelle gegenseitige Verschiebung oder Überdeckung der beiden Gitterlagen ohne Einfluss auf das Diffusionsverhalten der das Gitter durchströmenden Luft bleibt.

Ein weiterer Vorteil des beschriebenen Gasdetektors besteht darin, dass das mit Hilfe des Ringes 9 verschweisste Gitter M in der Kappe 6 wesentlich stabiler verankert ist als bei einer Schweissverbindung ohne Ring. Messungen zeigen, dass für das Herausschleudern des Gitters M aus der Kappe 6 mindestens die dreifache Kraft erforderlich ist wie bei einem konventionellen Gasdetektor.

Wie schon erwähnt wurde, kann der Gassensor 2 (Fig. 1) beispielsweise ein Halbleitersensor oder ein Brandgassensor oder auch ein optoakustischer Gassensor sein. Das verwendete Gitter M kann auch mehr als zwei Lagen aufweisen.

## Patentansprüche

1. Gasdetektor, mit einem Metallgehäuse (1), mit einem aufheizbaren Gassensor (2) und mit einer diesen abdeckenden Metallkappe (6) mit einem aus zwei Lagen (7, 8) bestehenden Metallgitter (M), welches den Gassensor gegen die Umgebung abschirmt, **dadurch gekennzeichnet, dass** an der dem Gassensor zugewandten Seite des Gitters dessen Rand durch einen Ring (9) abgedeckt und dass der Ring über das Gitter mit der Kappe verschweisst ist.

2. Gasdetektor nach Anspruch 1, **dadurch gekennzeichnet, dass** der Ring (9) aus dem gleichen Material wie das Gitter (M) besteht.

3. Gasdetektor nach Anspruch 2, **dadurch gekennzeichnet, dass** Ring (9) und Gitter (M) aus rostfreiem Stahl bestehen.

4. Gasdetektor nach einem der Ansprüche 1 bis 3, **dadurch gekennzeichnet, dass** der Ring (9) die Form einer Beilag- oder Unterlagscheibe aufweist.

5. Gasdetektor nach Anspruch 4, **dadurch gekennzeichnet, dass** der Gassensor (2) zum Nachweis von brennbaren Gasen ausgebildet ist.

6. Gasdetektor nach Anspruch 4, **dadurch gekennzeichnet, dass** der Gassensor (2) zum Nachweis von organischen Brandgasen und/oder Brandaerosolen ausgebildet ist.

7. Gasdetektor nach Anspruch 5, **dadurch gekennzeichnet, dass** der Gassensor (2) durch einen Halbleitersensor gebildet ist.

8. Gasdetektor nach einem der Ansprüche 1 bis 7, **dadurch gekennzeichnet, dass** das Gitter (M) mehr als zwei Lagen (7, 8) aufweist.

## Claims

1. A gas detector with a metal housing (1), a heatable gas sensor (2) and a metal cap (6) which covers the latter and has a metal grid (M) which consists of two layers (7, 8) and shields the gas sensor from the environment, **characterised in that** the edge of the grid is covered by a ring (9) on its side which faces the gas sensor, and the ring is welded to the cap via the grid.

2. A gas detector according to claim 1, **characterised in that** the ring (9) consists of the same material as the grid (M) .

3. A gas detector according to claim 2, **characterised in that** the ring (9) and the grid (M) consist of stainless steel.

4. A gas detector according to one of claims 1 to 3, **characterised in that** the ring (9) is in the form of a shim or washer.

5. A gas detector according to claim 4, **characterised in that** the gas sensor (2) is designed to detect combustible gases.

6. A gas detector according to claim 4, **characterised in that** the gas sensor (2) is designed to detect organic burnt gases and/or burnt aerosols.

7. A gas detector according to claim 5, **characterised in that** the gas sensor (2) is formed by a semiconductor sensor.

8. A gas detector according to one of claims 1 to 7, **characterised in that** the grid (M) comprises more than two layers (7, 8).

## Revendications

1. Détecteur de gaz comprenant un boîtier (1) métallique, un capteur (2) de gaz qui peut être chauffé et un chapeau (6) métallique le coiffant et ayant une grille (M) métallique qui est constituée de deux couches (7, 8) et qui protège le capteur de gaz de l'estérieur, **caractérisé en ce que**, du côté de la grille tournée vers le capteur de gaz, le bord de la grille est recouvert d'un anneau (9) et **en ce que** l'anneau est soudé au chapeau par la grille.

2. Détecteur de gaz suivant la revendication 1, **caractérisé en ce que** l'anneau (9) est en le même matériau que la grille (M).

3. Détecteur de gaz suivant la revendication 2, **caractérisé en ce que** l'anneau (9) et la grille (M) sont en acier inoxydable.

4. Détecteur de gaz suivant l'une des revendications 1 à 3, **caractérisé en ce que** l'anneau (9) a la forme d'une rondelle de calage ou d'une rondelle d'assise.

5. Détecteur de gaz suivant la revendication 4, **caractérisé en ce que** le capteur (2) de gaz est constitué pour détecter des gaz combustibles.

6. Détecteur de gaz suivant la revendication 4, **caractérisé en ce que** le capteur (2) de gaz est constitué pour détecter des gaz organiques d'incendie et/ou des aérosols d'incendie.

7. Détecteur de gaz suivant la revendication 5, **caractérisé en ce que** le capteur (2) de gaz est formé par un capteur à semi-conducteur.

8. Détecteur de gaz suivant l'une des revendications 1 à 7, **caractérisé en ce que** la grille (M) comporte plus de deux couches (7, 8).
